# EUROPEAN PATENT APPLICATION

(11) **EP 3 480 308 A1**
(43) Date of publication of application: **08.05.2019**
(21) Application number: 17819564.0
(22) Date of filing: 08.03.2017
(51) Int. Cl.: C12N 15/09, C12M 1/00, C12Q 1/68, G01N 33/53, G01N 37/00

(54) **COMPOSITION FOR PREVENTING DRYING OF GEL, GEL COMPOSITE AND DNA CHIP CONTAINING SAID COMPOSITE, AND METHOD FOR PRODUCING SAID COMPOSITION, COMPOSITE, AND CHIP**

(30) Priority: 29.06.2016 JP 2016129055
(71) Applicant: Mitsubishi Chemical Corporation, Tokyo 100-8251 (JP)
(72) Inventor: TOGAWA, Naoyuki, Tokyo 100-8251 (JP)
(74) Representative: TBK
(86) International application number: PCT/JP2017/010291
(87) International publication number: WO 2018/003195

(57) **Abstract**

The invention provides: a composition for preventing the drying of gel with which it is possible to prevent the drying of gel more easily than in the past; a gel complex and a DNA chip containing said composite; and a method for producing said composition, composite, and chip. This problem can be overcome by a composition for preventing the drying of gel that includes a polyhydric alcohol and at least one selected from gelatin, collagen, carrageenan, pectin, and agar.

SEQUENCE LISTING

<110>  Mitsubishi Rayon Co., Ltd.

<120>  Composition for preventing gel from drying, gel complex and DNA
       chip comprising the same, and production method thereof

<130>  G1543WO

<150>  JP 2016-129055
<151>  2016-06-29

<160>  14

<170>  PatentIn version 3.5

<210>  1
<211>  17
<212>  DNA
<213>  Artificial

<220>
<223>  synthetic DNA

<400>  1
ttggagctgg tggcgta                                                      17


<210>  2
<211>  17
<212>  DNA
<213>  Artificial

<220>
<223>  synthetic DNA

<400>  2
ttggagctag tggcgta                                                      17


<210>  3
<211>  17
<212>  DNA
<213>  Artificial

<220>
<223>  synthetic DNA

<400>  3
ttggagctcg tggcgta                                                      17


<210>  4
<211>  17
<212>  DNA
<213>  Artificial

<220>
<223>  synthetic DNA

<400>  4
ttggagcttg tggcgta                                                      17

<210>  5
<211>  17
<212>  DNA
<213>  Artificial

<220>
<223>  synthetic DNA

<400>  5
ttggagctga tggcgta                                                      17


<210>  6
<211>  17
<212>  DNA
<213>  Artificial

<220>
<223>  synthetic DNA

<400>  6
ttggagctgc tggcgta                                                      17


<210>  7
<211>  17
<212>  DNA
<213>  Artificial

<220>
<223>  synthetic DNA

<400>  7
ttggagctgt tggcgta                                                      17


<210>  8
<211>  21
<212>  DNA
<213>  Artificial

<220>
<223>  synthetic DNA

<400>  8
tggagctggt agcgtaggca a                                                 21


<210>  9
<211>  21
<212>  DNA
<213>  Artificial

<220>
<223>  synthetic DNA

<400>  9
tggagctggt cgcgtaggca a                                                 21


<210>  10
<211>  21

<212>  DNA
<213>  Artificial

<220>
<223>  synthetic DNA

<400>  10
tggagctggt tgcgtaggca a                                                21


<210>  11
<211>  21
<212>  DNA
<213>  Artificial

<220>
<223>  synthetic DNA

<400>  11
ggagctggtg acgtaggcaa g                                                21


<210>  12
<211>  21
<212>  DNA
<213>  Artificial

<220>
<223>  synthetic DNA

<400>  12
ggagctggtg ccgtaggcaa g                                                21


<210>  13
<211>  21
<212>  DNA
<213>  Artificial

<220>
<223>  synthetic DNA

<400>  13
ggagctggtg tcgtaggcaa g                                                21


<210>  14
<211>  30
<212>  DNA
<213>  Artificial

<220>
<223>  synthetic DNA

<400>  14
attagggtcg aacctacacg acaatgcacg                                       30

## Description

### TECHNICAL FIELD

The present invention relates to: a composition for preventing drying of a gel; a gel composite and a DNA chip containing said composite; and a method for producing said composition, composite and chip. The present invention further relates to a method for preventing drying of a DNA chip.

### BACKGROUND ART

Gels are materials which can hold a solvent whose weight is several hundred times to several thousand times the weight thereof and are conventionally utilized for high water absorption resins, disposable diapers, sanitary products, soft contact lenses, water-containing sheets for indoor planting, etc. Further, gels also have sustained-release property for drugs and are also applied to drug delivery systems and medical materials such as wound covering materials. Moreover, gels are also utilized for shock absorbing materials, vibration control/soundproofing materials, etc. Thus, gels are used for a wide range of applications.

Furthermore, gels are also used for the purpose of holding or coating a polynucleotide as a nucleic acid probe (sometimes referred to as just "probe") which is immobilized on a DNA chip (sometimes referred to as "DNA microarray") that is a tool for genetic analysis. The DNA chip is a useful device, wherein a DNA fragment having a sequence complementary to a nucleotide sequence of a gene is regularly aligned and immobilized on a substrate and a hybridization reaction with a nucleic acid base derived from the gene is performed on the substrate, thereby realizing utilization for gene expression analysis/polymorphism analysis, etc. As DNA chips, a DNA chip obtained by directly synthesizing a DNA on a substrate by utilizing the photolithographic technique (Patent Document 1, Patent Document 2), a DNA chip obtained by spotting a DNA on a slide glass by using a pin or the like (Non-Patent Document 1), etc. are known. Further, a DNA chip obtained by electrochemically immobilizing a DNA on a substrate (Patent Document 3), a through-hole type DNA chip, which is obtained by slicing a hollow fiber bundle obtained by bundling a plurality of hollow fibers in a direction intersecting with the longitudinal direction of the hollow fibers (Patent Document 4), etc. are also known.

However, since gels usually contain water as a solvent, when gels are under atmospheric environment, water gradually volatilizes and the gels may shrink and become cloudy. In particular, in the case of DNA chips in which gels are used, the gels deteriorate over time due to the influence of drying, and a hybridization reaction of a nucleic acid probe may be inhibited. Accordingly, in order to prevent drying of gels in DNA chips, a method of storing and transporting a DNA chip, wherein the DNA chip is immersed in a buffer solution and packaged individually, has been proposed (Patent Document 5).

### PRIOR ART DOCUMENTS

### PATENT DOCUMENTS

Patent Document 1: US Patent No. 5445934
Patent Document 2: US Patent No. 5774305
Patent Document 3: US Patent No. 5605662
Patent Document 4: International Publication WO01/098781 pamphlet
Patent Document 5: Japanese Laid-Open Patent Publication No. 2006-189307

### NON-PATENT DOCUMENTS

Non-Patent Document 1: Science 270, 467-470 (1995)

### SUMMARY OF THE INVENTION

### PROBLEMS TO BE SOLVED BY THE INVENTION

By storage and transport using an individual package as described above, drying of a gel can be prevented. However, at the time of use, it is required to unseal the individual package and to remove a buffer solution in the package before use, and therefore the procedure is complicated. Also in the case of a DNA chip, it is required to remove a filled buffer solution before using the DNA chip in a test, and in some cases, it is required to carry out washing at the time of removing the buffer solution, and therefore the procedure is complicated.

The purpose of the present invention is to provide: a composition for preventing drying of a gel with which it is possible to prevent drying of the gel more easily than before; a gel composite and a DNA chip containing said composite; and a method for producing said composition, composite and chip. Another purpose of the present invention is to provide a method for preventing drying of a DNA chip.

### MEANS FOR SOLVING THE PROBLEMS

The present invention was made in order to solve the above-described problems and has the below-described characteristics.
[1] A composition for preventing drying of a gel, comprising:
   a polyhydric alcohol; and
   at least one selected from gelatin, collagen, carrageenan, pectin and agar.
[2] The composition for preventing drying of the gel according to item [1], comprising the at least one selected from gelatin, collagen, carrageenan, pectin and agar in an amount of 1 to 10 parts by mass relative to 100 parts by mass of the polyhydric alcohol.
[3] The composition for preventing drying of the gel according to item [1] or [2], wherein the polyhydric alcohol is at least one selected from glycerol, diglycerol, pentaerythritol, trimethylolpropane, 1,2-propanediol, 1,3-propanediol, 1,2-butanediol, 1,3-butanediol, 1,4-butanediol, 1,4-pentanediol, 1,5-pentanediol, 1,6-hexanediol, 3,4-hexanediol, neopentyl glycol, diethylene glycol, triethylene glycol and dipropylene glycol.
[4] The composition for preventing drying of the gel according to any one of items [1] to [3], wherein the gel is a gel which a nucleic acid probe is held by or coated with on or in a substrate of a DNA chip.
[5] A gel composite, which comprises:
   a gel; and
   a gelled product of the composition for preventing drying of the gel according to any one of items [1] to [4].
[6] The gel composite according to item [5], wherein an interpenetrating network structure is formed at at least a part of the area between the gel and the gelled product of the composition for preventing drying of the gel.
[7] A method for producing a gel composite, which includes the steps of:
   applying the composition for preventing drying of the gel according to any one of items [1] to [4] to a gel or immersing the gel in the composition for preventing drying of the gel according to any one of items [1] to [4]; and
   gelling the composition for preventing drying of the gel.
[8] A method for producing a DNA chip comprising a gel composite, comprising the steps of:
   (i) three-dimensionally aligning a plurality of hollow fibers so that fiber axes of the hollow fibers are in the same direction and immobilizing the aligned hollow fibers with a resin to produce a hollow fiber bundle;
   (ii) introducing a gel precursor solution containing a nucleic acid probe into the hollow portion of each hollow fiber of the hollow fiber bundle;
   (iii) performing a reaction of the gel precursor solution introduced into the hollow portion of each hollow fiber of the hollow fiber bundle to hold a gel containing the nucleic acid probe in the hollow portion of each hollow fiber;
   (iv) slicing the hollow fiber bundle in a direction intersecting with the longitudinal direction of the hollow fibers to obtain the DNA chip;
   (v) applying the composition for preventing drying of the gel according to any one of items [1] to [4] to the DNA chip or the portion of the gel of the DNA chip, or immersing the DNA chip in the composition for preventing drying of the gel according to any one of items [1] to [4]; and
   (vi) gelling the composition for preventing drying of the gel.
[9] A DNA chip, having a nucleic acid probe held by or coated with a gel on the DNA chip,
   wherein the DNA chip comprises a gel composite comprising the gel and a gelled product of the composition for preventing drying of the gel according to any one of items [1] to [4].
[10] The DNA chip according to item [9], wherein the DNA chip is a through-hole type.
[11] The DNA chip according to item [9] or [10], wherein the DNA chip is a through-hole type with a hollow fiber.
[12] A method for preventing drying of a DNA chip, comprising the steps of:
   applying the composition for preventing drying of the gel according to any one of items [1] to [4] to the DNA chip or the portion of the gel of the DNA chip, or immersing the DNA chip in the composition for preventing drying of the gel according to any one of items [1] to [4]; and
   gelling the composition for preventing drying of the gel.

### ADVANTAGEOUS EFFECT OF THE INVENTION

According to the present invention, it is possible to provide: a composition for preventing drying of a gel with which it is possible to prevent drying of the gel more easily than before; a gel composite and a DNA chip containing said composite; and a method for producing said composition, composite and chip. Further, according to the present invention, it is also possible to provide a method for preventing drying of a DNA chip.

### BRIEF DESCRIPTION OF THE DRAWINGS

FIG. 1 shows photographs showing states of Samples 5-8 which were coated with silica gel and allowed to stand for 1 month.

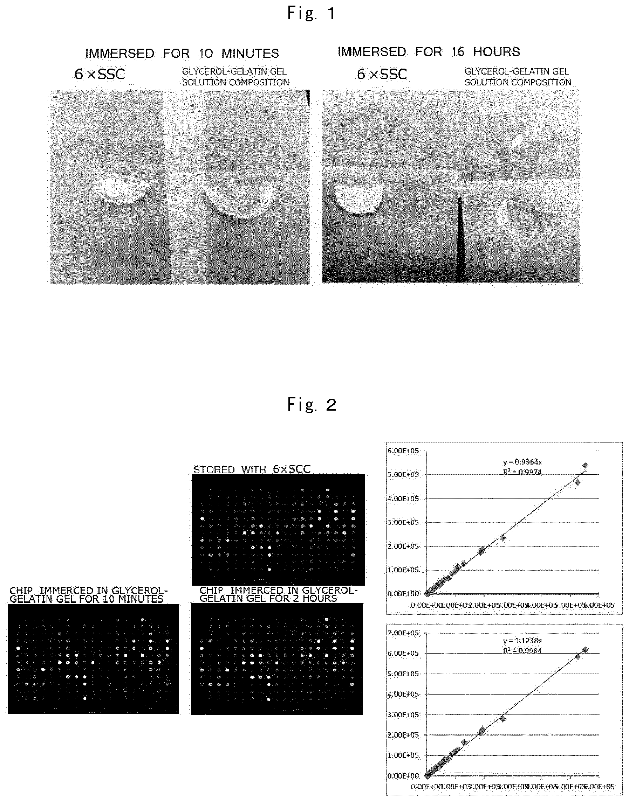

FIG. 2: 3 figures in the left part show comparison of results obtained by hybridizing aRNAs amplified from the mouse kidney respectively to DNA chips stored under 3 different conditions. The upper figure shows the result of a DNA chip stored using 6×SSC (Sample 11), the lower left figure shows the result of a DNA chip immersed in glycerol-gelatin gel for 10 minutes (Sample 9), and the lower right figure shows the result of a DNA chip immersed in glycerol-gelatin gel for 2 hours (Sample 10). Further, 2 graphs in the right part show the accuracy of judgment using chips. In the upper right graph, the vertical axis shows the result of Sample 11, and the horizontal axis shows the result of Sample 9. In the lower right graph, the vertical axis shows the result of Sample 11, and the horizontal axis shows the result of Sample 10.
FIG. 3 shows results obtained by carrying out hybridization to DNA chips for the detection of KRAS gene mutation after drying for 1 day. The left figure shows results of chips stored in a 6×SSC solution (Sample 14), and the right figure shows results of DNA chips, which were immersed in a glycerol-gelatin gel solution composition for 16 hours, then subjected to the step of draining off, and then subjected to a centrifuge for drying (Sample 13).
FIG. 4 shows results obtained by carrying out hybridization to DNA chips for the detection of KRAS gene mutation after drying for 1 month (Sample 15).
FIG. 5 shows results obtained by carrying out hybridization to DNA chips for the detection of KRAS gene mutation after drying for 5 months (Sample 16).

### DESCRIPTION OF THE EMBODIMENTS

Hereinafter, the present invention will be described in detail. The scope of the present invention is not limited to the description. In addition to the following examples, the present invention can be suitably changed and then practiced within a range in which the effects of the present invention are not reduced.

### [Composition for preventing drying of gel]

The composition for preventing drying of the gel according to the present invention (hereinafter sometimes referred to as "the composition according to the present invention") is a composition which comprises: a polyhydric alcohol; and at least one selected from gelatin, collagen, carrageenan, pectin and agar. A gelled product of a composition consisting of these raw materials can effectively prevent drying of the gel.

The composition ratio of the composition according to the present invention is not particularly limited, but at least one selected from gelatin, collagen, carrageenan, pectin and agar is contained in an amount of preferably 1 to 10 parts by mass, and more preferably 2 to 5 parts by mass relative to 100 parts by mass of the polyhydric alcohol.

The polyhydric alcohol to be used in the composition according to the present invention may have at least two hydroxyl groups per molecule. Accordingly, dihydric alcohols, trihydric alcohols, and polyhydric alcohols (higher than trihydric) can be used. Among them, glycerol, diglycerol, pentaerythritol, trimethylolpropane, 1,2-propanediol, 1,3-propanediol, 1,2-butanediol, 1,3-butanediol, 1,4-butanediol, 1,4-pentanediol, 1,5-pentanediol, 1,6-hexanediol, 3,4-hexanediol, neopentyl glycol, diethylene glycol, triethylene glycol and dipropylene glycol are preferred, and glycerol is particularly preferred.

In the composition according to the present invention, at least one selected from gelatin, collagen, carrageenan, pectin and agar is contained. Preferably, at least one selected from gelatin and collagen is contained in the composition.

In the present invention, examples of preferred compositions include a composition comprising gelatin and glycerol, a composition comprising gelatin and diglycerol, a composition comprising gelatin and pentaerythritol, a composition comprising gelatin and trimethylolpropane, a composition comprising gelatin and 1,2-propanediol, a composition comprising gelatin and 1,3-propanediol, a composition comprising gelatin and 1,2-butanediol, a composition comprising gelatin and 1,3-butanediol, a composition comprising gelatin and 1,4-butanediol, a composition comprising gelatin and 1,4-pentanediol, a composition comprising gelatin and 1,5-pentanediol, a composition comprising gelatin and 1,6-hexanediol, a composition comprising gelatin and 3,4-hexanediol, a composition comprising gelatin and neopentyl glycol, a composition comprising gelatin and diethylene glycol, a composition comprising gelatin and triethylene glycol and a composition comprising gelatin and dipropylene glycol, and more preferred is a composition comprising gelatin and glycerol.

Moreover, in the present invention, examples of preferred compositions include a composition comprising collagen and glycerol, a composition comprising collagen and diglycerol, a composition comprising collagen and pentaerythritol, a composition comprising collagen and trimethylolpropane, a composition comprising collagen and 1,2-propanediol, a composition comprising collagen and 1,3-propanediol, a composition comprising collagen and 1,2-butanediol, a composition comprising collagen and 1,3-butanediol, a composition comprising collagen and 1,4-butanediol, a composition comprising collagen and 1,4-pentanediol, a composition comprising collagen and 1,5-pentanediol, a composition comprising collagen and 1,6-hexanediol, a composition comprising collagen and 3,4-hexanediol, a composition comprising collagen and neopentyl glycol, a composition comprising collagen and diethylene glycol, a composition comprising collagen and triethylene glycol and a composition comprising collagen and dipropylene glycol, and more preferred is a composition comprising collagen and glycerol.

The composition according to the present invention may further comprise water if desired. When using the composition according to the present invention in a DNA chip, from the viewpoint of minimizing the influence of impurities on analysis, water to be used is preferably ultrapure water, pure water or distilled water, and particularly preferably ultrapure water. As ultrapure water, for example, Milli-Q water can be used. The content of water is not particularly limited, but is preferably 10 to 50% by mass, and more preferably 20 to 40% by mass relative to the total amount of the composition for preventing drying of the gel. Meanwhile, after the treatment, the water content may become 1% by mass or less due to evaporation.

The composition according to the present invention may contain various additives. Examples of such additives include, but are not limited to, an antioxidant, a preservative agent and an ultraviolet absorber. The amount of the additives is preferably less than 3% by mass, and more preferably less than 1% by mass based on the total amount of the composition for preventing drying of the gel.

The type of the gel, drying of which is prevented by the composition according to the present invention, is not limited, and the gel may be either a physical gel or a chemical gel, and may be either a gel to be used for food applications, a gel to be used for pharmaceutical applications, or a gel to be used for other applications such as industrial applications and agricultural applications.

Among them, the composition according to the present invention can be preferably used for gels whose function and effects are attenuated or reduced by drying. Among them, for example, a gel which holds or coats a nucleic acid probe on or in a substrate of a DNA chip is more preferred. The degree of the influence of the gelled product of the composition for preventing drying of the gel according to the present invention on analyses is very small or ignorable, and for this reason, analysis of a DNA chip can be conducted without removing the product. Meanwhile, even in the case where the gelled product is dissolved and removed by heating the DNA chip in a process of hybridization, cleaning or the like, the DNA chip can be analyzed without any problems.

The gel which holds or coats a nucleic acid probe is preferably a product obtained by mixing a gel precursor solution with a nucleic acid probe and then performing a polymerization reaction for gelation. Examples of the gel precursor solution include those containing at least one monomer such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethyl methacrylate, (meth)acrylic acid and allyl dextrin, and as a crosslinkable monomer, methylenebis (meth)acrylamide, polyethylene glycol di(meth)acrylate or the like. Further, according to need, a polymerization initiator (radical polymerization initiator, cationic polymerization initiator, anionic polymerization initiator, etc.) and a solvent (water, organic solvent, etc.) and the like are added to the gel precursor solution.

The composition according to the present invention is preferably solated when flowability is increased by heating or the like. By solation of the composition according to the present invention, it can be applied to a gel or DNA chip, or the gel or DNA chip can be immersed in the composition. Further, it is preferred that by solation, the composition according to the present invention penetrates a network structure of the gel (primary network structure) and then gelled to form a secondary network structure, thereby forming an interpenetrating network structure, wherein the two network structures are intertangled between the gel and the composition according to the present invention. It is considered that drying of the gel can be more effectively prevented by forming the interpenetrating network structure.

The method for producing the composition according to the present invention is not particularly limited, and the composition can be produced by a publicly-known method. For example, the composition can be produced by a general solution mixing method in which raw materials are dissolved in water to be mixed together. Further, heating may be performed in manufacturing processes according to need. Heating can be performed at a temperature enough for promoting dissolution of raw materials. For example, heating can be performed at 30 to 80°C, preferably 40 to 70°C, and more preferably 45 to 65°C.

As described above, the gelled product of the composition according to the present invention can prevent drying of every gel, and can also be used for preventing drying of a gel which holds or coats a nucleic acid probe on or in a substrate of a DNA chip. In this regard, the DNA chip is a device, in which a DNA fragment having a sequence complementary to a nucleotide sequence of a gene (nucleic acid probe) is regularly aligned and immobilized on a substrate and a hybridization reaction with a nucleic acid base derived from the gene is performed on the substrate, thereby realizing utilization for gene analysis or assay for diagnosis or the like. The DNA chip may also be referred to as a "DNA microarray". There are various types of DNA chips including: a DNA chip obtained by directly synthesizing a DNA on a substrate by utilizing the photolithographic technique; a DNA chip obtained by spotting a DNA on a slide glass by using a pin or the like; a DNA chip obtained by electrochemically immobilizing a DNA on a substrate; and a through-hole type DNA chip, which is obtained by slicing a hollow fiber bundle obtained by bundling a plurality of hollow fibers (tubular bodies) in a direction intersecting with the longitudinal direction of the hollow fibers. The composition for preventing drying of the gel according to the present invention can be used for any type of DNA chip.

### [Gel composite]

The gel composite according to the present invention comprises a gel and a gelled product of the composition according to the present invention. The embodiment of the gel composite according to the present invention is not particularly limited as long as the gel and the gelled product of the composition according to the present invention are included. For example, an embodiment in which a part of the gel is coated with the gelled product of the composition according to the present invention is also included in the embodiment of the gel composite according to the present invention. Further, in the gel composite according to the present invention, an interpenetrating network structure is preferably formed at at least a part of the area between the gel and the gelled product of the composition according to the present invention. It is considered that drying of the gel can be more effectively prevented by forming the interpenetrating network structure.

Examples of the gel to be used in the gel composite according to the present invention include various gels as described above, but a gel which holds or coats a probe on or in a substrate of a DNA chip is preferred. Specifically, a gel obtained by mixing a gel precursor solution with a nucleic acid probe and then performing a polymerization reaction for gelation is preferred. Examples of the gel precursor solution include those containing at least one monomer such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethyl methacrylate, (meth)acrylic acid and allyl dextrin, and as a crosslinkable monomer, methylenebis (meth)acrylamide, polyethylene glycol di(meth)acrylate or the like. Further, according to need, a polymerization initiator (radical polymerization initiator, cationic polymerization initiator, anionic polymerization initiator, etc.) and a solvent (water, organic solvent, etc.) and the like are added to the gel precursor solution.

The gel composite according to the present invention can be produced by a method including the steps of: applying the composition for preventing drying of the gel according to the present invention to the gel, or immersing the gel in the composition for preventing drying of the gel according to the present invention; and gelling the composition for preventing drying of the gel. It is preferred that the composition for preventing drying of the gel according to the present invention penetrates a network structure of the gel (primary network structure) and then gelled to form a secondary network structure, thereby forming an interpenetrating network structure, wherein the two network structures are intertangled between the gel and the composition for preventing drying of the gel according to the present invention. It is considered that drying of the gel can be more effectively prevented by forming the interpenetrating network structure.

If desired, the method for producing the gel composite according to the present invention may include a step of solating the composition for preventing drying of the gel before the step of applying the composition for preventing drying of the gel according to the present invention to the gel or immersing the gel in the composition for preventing drying of the gel according to the present invention. Solation can be performed, for example, by heating.

The time for immersing the gel in the composition for preventing drying of the gel according to the present invention is not particularly limited, and it is sufficient when the gel composite according to the present invention is formed at at least a part of the gel, drying of which is to be prevented (for example, to the extent that drying of the gel can be prevented by coating the surface of the gel with the composition for preventing drying of the gel according to the present invention). The time for immersion can be suitably selected depending on the type and size of the gel, drying of which is to be prevented, the type of the composition for preventing drying of the gel, etc. The time may be, for example, 1 minute or more, preferably 10 minutes or more, and more preferably 1 hour or more. Moreover, the upper limit of the time for immersion is not limited, and it is sufficient when the gel composite according to the present invention is formed throughout the gel, drying of which is to be prevented.

The method for immersion is not limited. The gel may be immersed in the composition for preventing drying of the gel while stirring, or the gel may be immersed in the composition for preventing drying of the gel, followed by stirring, or the gel may be immersed in the composition for preventing drying of the gel, followed by shaking the gel.

When applying the composition for preventing drying of the gel according to the present invention to the gel, various publicly-known methods can be used. For example, the composition can be applied by using a brush or the like, or a spray coat method, a bar coat method, a spin coat method, a flow coat method or the like can be used.

### [DNA chip including gel composite]

The DNA chip according to the present invention is a DNA chip having a nucleic acid probe held by or coated with a gel on the DNA chip, and the DNA chip includes a gel composite comprising a gel and a gelled product of the composition for preventing drying of the gel according to the present invention. According to one embodiment of the present invention, in a through-hole type DNA chip, which is obtained by slicing a hollow fiber bundle obtained by bundling a plurality of hollow fibers in a direction intersecting with the longitudinal direction of the hollow fibers, a nucleic acid probe is held by a gel in the hollow portion of each hollow fiber, and a gel composite is formed by at least a part of the gel held in the hollow portion and a gelled product of the composition according to the present invention. Since the DNA chip according to the present invention includes the gel composite comprising the gel and the gelled product for preventing drying of the gel according to the present invention, drying of the gel which holds or coats the nucleic acid probe immobilized on the DNA chip can be effectively prevented. Moreover, the degree of the influence of the gelled product of the composition according to the present invention on analyses is very small or ignorable, and for this reason, analysis of a DNA chip can be conducted without removing the product. Meanwhile, even in the case where the gelled product is dissolved and removed by heating the DNA chip in a process of hybridization, cleaning or the like, the DNA chip can be analyzed without any problems.

Further, according to a preferred embodiment of the present invention, in a through-hole type DNA chip, an interpenetrating network structure is formed between the gel held in the hollow portion of the hollow fiber and the gelled product of the composition according to the present invention. As used herein, the "interpenetrating network structure" refers to a structure, in which a primary network structure and a secondary network structure are overlapped and intertangled in a gel comprising the first gel having a primary network structure and the second gel having a secondary network structure. Further, a gel having such a structure is called a "double network gel". It is considered that drying of the gel can be more effectively prevented by forming the interpenetrating network structure.

In this specification, "to hold or coat a probe on or in a substrate of a DNA chip" includes an embodiment in which a probe synthesized or immobilized on a substrate of a DNA chip is coated with a gel and an embodiment in which a gel is held by a probe in the hollow portion of a hollow fiber in a substrate of a DNA chip. Further, "to coat" includes not only an embodiment in which a probe is coated in a manner such that the entire probe is enclosed, but also an embodiment in which at least a part of a probe is coated.

There are various types of DNA chips as described above, and any type of DNA chip can be used in the present invention. Among them, a through-hole type DNA chip is preferred. Examples of the through-hole type DNA chip include: a through-hole type DNA chip, which is obtained by slicing a hollow fiber bundle obtained by bundling a plurality of hollow fibers in a direction intersecting with the longitudinal direction of the hollow fibers; and a through-hole type DNA chip having through holes formed with a porous body such as a porous inorganic compound including aluminium oxide. The DNA chip obtained by using a plurality of hollow fibers has a plurality of spots (sliced hollow fibers, hereinafter also referred to as the "through holes"), the gel is held in the spots, and the nucleic acid probe is held in the gel. Similarly, in the case of the through-hole type DNA chip having through holes formed with a porous body, the gel is held in the through holes and the nucleic acid probe is held in the gel. Since the gel in through holes usually contains water as a solvent, when the through-hole type DNA chip is under atmospheric environment, water gradually volatilizes and the gel deteriorates over time, and as a result, a hybridization reaction of the nucleic acid probe may be inhibited and the gel may drop off from through holes. However, when using the through-hole type DNA chip according to the present invention, such time-dependent deterioration can be effectively prevented, and inhibition of the hybridization reaction and dropping of the gel can be prevented.

The nucleic acid probe is a nucleic acid to be used for the detection of a deoxyribonucleic acid (DNA), a ribonucleic acid (RNA) or the like. Further, analogs of nucleic acids such as a peptide nucleic acid (PNA) are also included in the nucleic acid probe. These may be synthesized or prepared from an organism.

The nucleic acid probe may be held by the gel. The gel to be used for holding the nucleic acid probe is preferably a product obtained by mixing a gel precursor solution with a nucleic acid probe and then performing a polymerization reaction for gelation. Examples of the gel precursor solution include those containing at least one monomer such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethyl methacrylate, (meth)acrylic acid and allyl dextrin, and as a crosslinkable monomer, methylenebis (meth)acrylamide, polyethylene glycol di(meth)acrylate or the like. Further, according to need, a polymerization initiator (radical polymerization initiator, cationic polymerization initiator, anionic polymerization initiator, etc.) and a solvent (water, organic solvent, etc.) and the like are added to the gel precursor solution.

The thickness of one DNA chip according to the present invention is not particularly limited, and for example, it is preferably 1 µm to 10,000 µm, and more preferably 100 µm to 5,000 µm.

As the DNA chip to be used in the present invention, general commercially-available products may be used. Examples of such commercially-available DNA chips include Genopal (registered trademark) manufactured by Mitsubishi Rayon Co., Ltd. and CodeLink chip (registered trademark) which is coated with gel.

The DNA chip can be produced by various publicly-known methods. For example, a through-hole type DNA chip can be produced by the method described in Patent Document 5 (Japanese Laid-Open Patent Publication No. 2006-189307) or the like.

### [Method for producing DNA chip including gel composite]

According to one embodiment of the present invention, a method for producing a DNA chip including a gel composite (hereinafter sometimes referred to as "the production method according to the present invention") is provided. Specifically, a DNA chip comprising a gel composite can be produced by a method comprising the below-described steps:
(i) three-dimensionally aligning a plurality of hollow fibers so that fiber axes of the hollow fibers are in the same direction and immobilizing the aligned hollow fibers with a resin to produce a hollow fiber bundle;
(ii) introducing a gel precursor solution containing a nucleic acid probe into the hollow portion of each hollow fiber of the hollow fiber bundle;
(iii) performing a reaction of the gel precursor solution introduced into the hollow portion of each hollow fiber of the hollow fiber bundle to hold a gel containing the nucleic acid probe in the hollow portion of each hollow fiber;
(iv) slicing the hollow fiber bundle in a direction intersecting with the longitudinal direction of the hollow fibers to obtain the DNA chip;
(v) applying the composition for preventing drying of the gel according to the present invention to the DNA chip or the portion of the gel of the DNA chip, or immersing the DNA chip in the composition for preventing drying of the gel according to the present invention; and
(vi) gelling the composition for preventing drying of the gel according to the present invention.

In this regard, according to need, a step of solating the composition for preventing drying of the gel according to the present invention by heating may be added to the method (before step (v)). The specific heating temperature can be suitably selected depending on raw materials of the composition for preventing drying of the gel to be used. For example, when using gelatin or collagen as a raw material of the composition for preventing drying of the gel, the composition can be sufficiently solated by heating to about 45°C to 70°C. Even in the case of other raw materials, the heating temperature can be suitably set by those skilled in the art.

After the DNA chip is obtained by step (iv), the DNA chip is immersed in the composition for preventing drying of the gel, or the composition for preventing drying of the gel is applied to the DNA chip. In the case of immersion, it is not required that the entire DNA chip is completely immersed in the composition for preventing drying of the gel, and it is sufficient when at least a nucleic acid probe, at least the surface of a gel held in the hollow portion, or at least an area where a nucleic acid probe is arranged and immobilized is immersed in the composition for preventing drying of the gel.

The time for immersion is not particularly limited, and it is sufficient when the gel composite according to the present invention is formed at at least a part of the gel in the DNA chip (for example, to the extent that drying of the gel can be prevented by coating the surface of the gel with the composition for preventing drying of the gel according to the present invention). The time for immersion can be suitably selected depending on the type and size of the gel, drying of which is to be prevented, the type of the composition for preventing drying of the gel, etc. The time may be, for example, 1 minute or more, preferably 10 minutes or more, and more preferably 1 hour or more. Moreover, the upper limit of the time for immersion is not limited, and it is sufficient when the gel composite according to the present invention is formed throughout the gel.

The method for immersion is not limited. The gel may be immersed in the composition for preventing drying of the gel while stirring, or the gel may be immersed in the composition for preventing drying of the gel, followed by stirring, or the gel may be immersed in the composition for preventing drying of the gel, followed by shaking the gel.

When applying the composition for preventing drying of the gel according to the present invention to the DNA chip, various publicly-known methods can be used. For example, the composition can be applied by using a brush or the like, or a spray coat method, a bar coat method, a spin coat method, a flow coat method or the like can be used.

It is preferred that after immersion or application of step (v), a step of draining off is carried out to remove an excess of the composition for preventing drying of the gel. The step of draining off can be carried out by various publicly-known methods, but it is preferably carried out by the centrifugation treatment, and from the viewpoint of promoting efficiency, it is more preferred that the centrifugation treatment is carried out directly for a plate with a plurality of DNA chips being stood thereon.

The step (vi) of gelling the composition for preventing drying of the gel according to the present invention is not particularly limited, but for example, can be carried out by drying by means of the centrifugation treatment, cooling of the composition for preventing drying of the gel, or the like. It is preferred that in step (v), the composition for preventing drying of the gel according to the present invention penetrates a network structure of the gel (primary network structure) and then in step (vi), the composition for preventing drying of the gel is gelled to form a secondary network structure, thereby forming an interpenetrating network structure, wherein the two network structures are intertangled between the gel and the composition for preventing drying of the gel according to the present invention. It is considered that drying of the gel can be more effectively prevented by forming the interpenetrating network structure.

### [Method for preventing drying of DNA chip]

According to one embodiment of the present invention, a method for preventing drying of a DNA chip by using a composition for preventing drying of a gel is provided. Specifically, drying of a gel which holds or coats a nucleic acid probe immobilized on a DNA chip can be prevented by a method, which includes the steps of: applying the composition for preventing drying of a gel according to the present invention to the DNA chip or the portion of the gel of the DNA chip, or immersing the DNA chip in the composition for preventing drying of the gel according to the present invention; and gelling the composition for preventing drying of the gel according to the present invention.

### EXAMPLES

Hereinafter, the present invention will be more specifically described by way of examples. The scope of the present invention is not limited to the description. In addition to the following examples, the present invention can be suitably changed and then practiced within a range in which the effects of the present invention are not reduced.

### Example 1

### 1. Preparation of gel for diffusing and immersing nucleic acid probe

Firstly, a gel to be used for diffusing and immersing a nucleic acid probe was prepared. The raw materials used in the preparation and the amounts thereof are shown in tables below.

**Table 1a**

| Monomer solution | | |
|---|---|---|
| DMAAm [g] | MBAAm [g] | Ultrapure water [g] |
| 5.13 | 0.57 | 34.05 |

**Table 1b**

| Polymerization initiator solution | |
|---|---|
| VA-044 [g] | Ultrapure water [g] |
| 1.00 | 9.00 |

**Table 1c**

| Polymerization solution | | | |
|---|---|---|---|
| Glycerol [g] | Monomer solution [g] | Polymerization initiator solution [g] | Total [g] |
| 7.60 | 4.24 | 0.16 | 12.00 |

In the tables, DMAAm represents N,N-dimethylacrylamide (manufactured by Sigma-Aldrich), MBAAm represents N,N-methylenebisacrylamide (manufactured by Sigma-Aldrich), VA-044 represents 2,2'-bis(2-imidazolin-2-yl)[2,2'-azobispropane] dihydrochloride (manufactured by Wako Pure Chemical Industries, Ltd.), and Ultrapure water represents Milli-Q water.

Firstly, a monomer solution and a polymerization initiator solution were respectively prepared with blending amounts shown in Table 1a and Table 1b. Secondly, these solutions were weighed to be in amounts described in Table 1c, and mixed with glycerol to prepare a polymerization solution of Table 1c. To 12 g of this polymerization solution, 4 g of ultrapure water was added and mixed together, and the mixture was deaerated and put into a 12 mL glass bottle to be allowed to stand, thereby preparing about 3.8% by mass of N,N-dimethylacrylamide gel.

### 2. Preparation of composition for preventing drying of gel

19.2 g of glycerol (for fluorometric analysis, manufactured by Kanto Chemical Co., Inc.), 10 g of ultrapure water (Milli-Q water) and 0.60 g of powdered gelatin (collagen content: 4.45 g/5 g, selling agency: EIGHT CO-OPERATIVE BUYING CO., LTD) were mixed together at room temperature. After that, the mixture was transferred to a thermostatic bath at 60°C and warmed for about 1 hour while stirring suitably, and the powdered gelatin was completely dissolved, thereby obtaining a composition for preventing drying of a gel (hereinafter also referred to as the "glycerol-gelatin gel solution composition"). The obtained glycerol-gelatin gel solution composition was kept at 60°C.

### 3. Evaluation of composition for preventing drying of gel

Subsequently, the chip gel of about 3.8% by mass of N,N-dimethylacrylamide gel prepared in the above-described step 1 was cut in half in the center of the round shape using a spatula. The cut chip gel was transferred to a brand-new 25 mL flat bottom tube in a manner such that the chip gel did not lose its shape. The same operation was repeated to prepare 8 samples in total.

Among the obtained samples, to 4 samples, 6 mL of the glycerol-gelatin gel solution composition prepared in the above-described step 2 was added, and to the remaining 4 samples, 6 mL of a 6×SSC solution (aqueous solution containing 99.9 mM of sodium chloride and 99.9 mM of sodium citrate dihydrate; pH=7.0) was added, and the chip gels of about 3.8% by mass of N,N-dimethylacrylamide gel prepared in the above-described step 1 were respectively immersed therein. Among the 4 samples immersed in the 6×SSC solution, 2 samples were immersed at 60°C for 10 minutes, and the remaining 2 samples were immersed at 60°C for 16 hours. The same operation was carried out for the samples immersed in the glycerol-gelatin gel solution composition.

### 3-1. Evaluation by means of storage in refrigerator

The samples immersed in the glycerol-gelatin gel solution composition at 60°C for 10 minutes (Sample 1) and at 60°C for 16 hours (Sample 2), and the samples immersed in the 6×SSC solution at 60°C for 10 minutes (Sample 3) and at 60°C for 16 hours (Sample 4) were subjected to the step of draining off. After that, the flat bottom tubes with the samples put therein were covered with a Parafilm with an air hole made and allowed to stand in a refrigerator at 4°C for 3 months. As a result, all the chip gels immersed in the 6×SSC solution (Sample 3 and Sample 4) shrank to about half the size, but those immersed in the glycerol-gelatin gel solution composition (Samples 1 and 2) had almost the same size as the original size.

### 3-2. Evaluation by means of storage in desiccant

Evaluation was further carried out by using Samples 5-8 (samples prepared in manners similar to those for Samples 1-4 and not used for the evaluation 3-1). Firstly, a cooking sheet (manufactured by Asahi Kasei Home Products Corporation) was cut to prepare rectangular pieces (about 5 cm×10 cm). These pieces were folded in half, in which the chip gels of Samples 5-8 were respectively sandwiched, and coated with silica gel and allowed to stand for 1 month.

As a result, the chip gels immersed in the 6×SSC solution (Samples 7 and 8) became cloudy and shrank, but those immersed in the glycerol-gelatin gel solution composition (Samples 5 and 6) kept the original shape. The results are shown in Figure 1.

### Example 2

A through-hole type DNA chip having about 3.8% by mass of N,N-dimethylacrylamide gel as a spot was prepared in a manner similar to that described in Japanese Laid-Open Patent Publication No. 2006-189307. As a probe of the DNA chip, a mouse-version probe of an anti-aging chip manufactured by Mitsubishi Rayon Co., Ltd. was used.

For immersing the DNA chip in the glycerol-gelatin gel solution composition, a container for draining off, in which a plurality of DNA chips are stood and housed, and which enables draining off by means of a centrifuge for a whole plate, was prepared.

### <Preparation of chip treated with glycerol-gelatin gel solution composition>

About 50 mL of a glycerol-gelatin gel solution composition having the same composition as that in Example 1 was prepared. It was poured into the container as shown in Figure 8, and a portion of the DNA chip including an area in which a gel with a nucleic acid probe being aligned and immobilized thereon exists was immersed at 60°C for a predetermined amount of time. After that, the container was tilted to remove the glycerol-gelatin gel solution composition, and further, the container was directly subjected to a centrifuge to remove an excess of the gel solution composition, and the DNA chip became in the dry state. After that, it was put into a bag together with silica gel and the bag was sealed, and it was stored in a dark place at room temperature until it was used.

The above-described operation was carried out with different immersion times to obtain Samples 9 and 10 described below. Further, Sample 11 described below was prepared for comparison.
(9) DNA chip which was immersed in the glycerol-gelatin gel solution composition for 10 minutes and then left in the dry state overnight (mouse-version probe of anti-aging chip manufactured by Mitsubishi Rayon Co., Ltd.)
(10) DNA chip which was immersed in the glycerol-gelatin gel solution composition for 120 minutes and then left in the dry state overnight (mouse-version probe of anti-aging chip manufactured by Mitsubishi Rayon Co., Ltd.)
(11) DNA chip which was stored in a state where it was immersed in the 6×SSC solution (mouse-version probe of anti-aging chip manufactured by Mitsubishi Rayon Co., Ltd.)

To the above-described 3 types of DNA chips, aRNAs amplified from the mouse kidney were hybridized to compare results thereof.
The hybridization conditions were as follows:
0.12M TNT buffer, 65°C, 16 hours
The conditions for washing after hybridization were as follows:
6 ml of 0.12M TNT buffer, 65°C, immersed for 20 minutes, allowed to stand twice;
6 ml of 0.12M TN buffer, 65°C, immersed for 10 minutes, allowed to stand once

The results are shown in Figure 2. In this regard, when Sample 9 was compared to Sample 11 and Sample 10 was compared to Sample 11, the correlation coefficients were respectively 0.9974 and 0.9984. It is understood from this point that almost the same result was obtained with respect to the DNA chip, which was treated with the glycerol-gelatin gel solution composition and dried, and the DNA chip, which was stored in a state where it was immersed in the 6×SSC solution and was not dried.

### Example 3

DNA chips for judging single nucleotide mutation of the KRAS gene were prepared. 14 types of sequences shown in the table below were used for probes.

**Table 2**

| | | |
|---|---|---|
| WT | TTGGAGCTGGTGGCGTA | SEQ ID NO: 1 |
| mt1 | TTGGAGCTAGTGGCGTA | SEQ ID NO: 2 |
| mt2 | TTGGAGCTCGTGGCGTA | SEQ ID NO: 3 |
| mt3 | TTGGAGCTTGTGGCGTA | SEQ ID NO: 4 |
| mt4 | TTGGAGCTGATGGCGTA | SEQ ID NO: 5 |
| mt5 | TTGGAGCTGCTGGCGTA | SEQ ID NO: 6 |
| mt6 | TTGGAGCTGTTGGCGTA | SEQ ID NO: 7 |
| mt7 | TGGAGCTGGTAGCGTAGGCAA | SEQ ID NO: 8 |
| mt8 | TGGAGCTGGTCGCGTAGGCAA | SEQ ID NO: 9 |
| mt9 | TGGAGCTGGTTGCGTAGGCAA | SEQ ID NO: 10 |
| mt10 | GGAGCTGGTGACGTAGGCAAG | SEQ ID NO: 11 |
| mt1 1 | GGAGCTGGTGCCGTAGGCAAG | SEQ ID NO: 12 |
| mt12 | GGAGCTGGTGTCGTAGGCAAG | SEQ ID NO: 13 |
| N.C. | ATTAGGGTCGAACCTACACGACAATGCACG | SEQ ID NO: 14 |

Regarding the DNA chips used in Example 3, the conditions for hybridization and washing were optimized by using a specimen in which a complementary oligo DNA was fluorescently labeled in advance and a specimen in which PCR was carried out from a template mixed in a model-based manner.
The hybridization conditions were as follows:
0.12M TNT buffer, 50°C, 30 minutes
The washing conditions were as follows:
6 ml of 0.12M TNT buffer, 50°C, immersed for 10 minutes, allowed to stand twice;
6 ml of 0.12M TN buffer, 50°C, immersed for 10 minutes, allowed to stand once

The obtained DNA chips were immersed in the glycerol-gelatin gel solution composition for 10 minutes or for 16 hours in a manner similar to that in Example 2, then subjected to the step of draining off and subjected to a centrifuge, thereby preparing DNA chips in the dry state (Sample 12 and Sample 13).

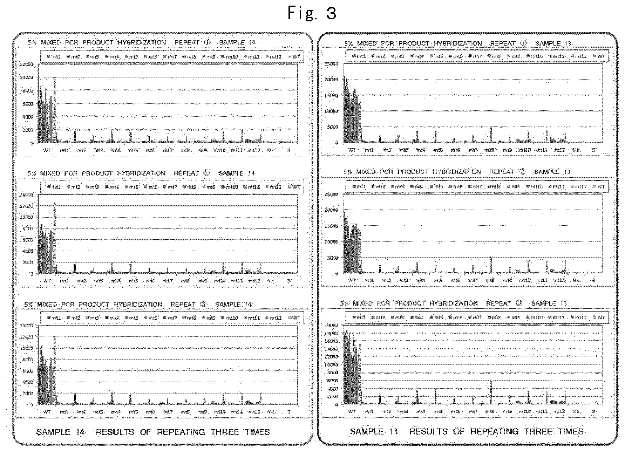

When a specimen obtained by carrying out PCR from a model specimen in which 5% of a mutant-type template was mixed was hybridized to the DNA chips of Sample 12 and Sample 13 one day after drying and the DNA chip stored in the 6×SSC solution (Sample 14), accurate judgment was successfully made for the DNA chips of Sample 12 and Sample 13 after drying as in the case of the DNA chip of Sample 14. The results of Samples 13 and 14 are shown in Figure 3.

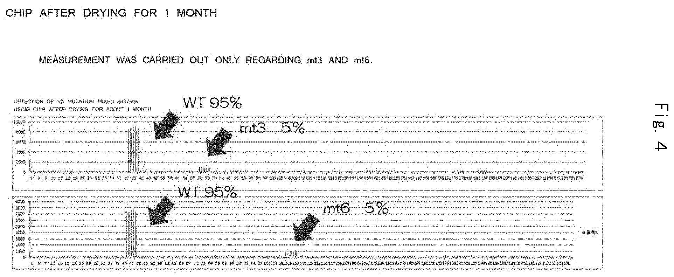

When a specimen obtained by carrying out PCR from a model specimen in which a 5% mutation-type template of mutation 3 (mt3) or mutation 6 (mt6) was mixed was hybridized to the chip of Sample 13 which was further stored for 1 month (Sample 15) and the chip stored in the 6×SSC solution (Sample 14), accurate judgment was also successfully made for the chip after drying for 1 month (Sample 15). The results are shown in Figure 4.

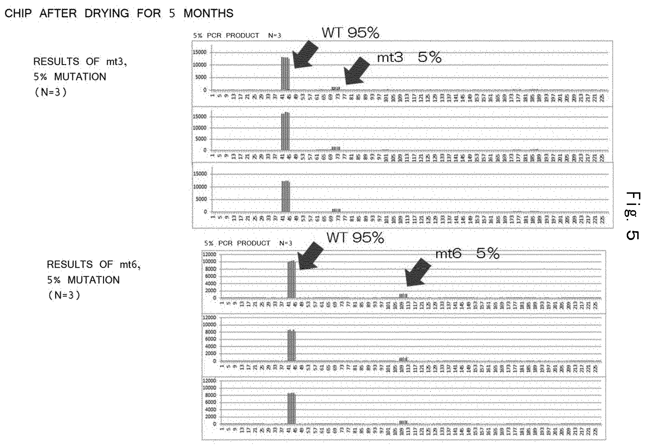

When a specimen obtained by carrying out PCR from a model specimen in which a 5% mutation-type template of mutation 3 (mt3) or mutation 6 (mt6) was mixed was hybridized to the chip which was further stored for 5 months (Sample 16) and the chip stored in the 6×SSC solution (Sample 14), accurate judgment was also successfully made for the chip after drying for 5 months (Sample 16). The results are shown in Figure 5.

### Sequence Listing Free Text

SEQ ID NOs: 1 to 14: synthetic DNAs

## Claims

1. A DNA chip, having a nucleic acid probe held by or coated with a gel on the DNA chip,
wherein the DNA chip comprises a gel composite comprising the gel and a gelled product of a composition for preventing drying of the gel,
wherein the composition for preventing drying of the gel comprises:
a polyhydric alcohol; and
at least one selected from gelatin, collagen, carrageenan, pectin and agar.

2. The DNA chip according to claim 1, wherein the composition for preventing drying of the gel comprises the at least one selected from gelatin, collagen, carrageenan, pectin and agar in an amount of 1 to 10 parts by mass relative to 100 parts by mass of the polyhydric alcohol.

3. The DNA chip according to claim 1 or 2, wherein the polyhydric alcohol is at least one selected from glycerol, diglycerol, pentaerythritol, trimethylolpropane, 1,2-propanediol, 1,3-propanediol, 1,2-butanediol, 1,3-butanediol, 1,4-butanediol, 1,4-pentanediol, 1,5-pentanediol, 1,6-hexanediol, 3,4-hexanediol, neopentyl glycol, diethylene glycol, triethylene glycol and dipropylene glycol.

4. The DNA chip according to any one of claims 1 to 3, wherein the DNA chip is a through-hole type.

5. The DNA chip according to any one of claims 1 to 4, wherein the DNA chip is a through-hole type with a hollow fiber.

6. A method for producing a DNA chip comprising a gel composite, comprising the steps of:
(i) three-dimensionally aligning a plurality of hollow fibers so that fiber axes of the hollow fibers are in the same direction and immobilizing the aligned hollow fibers with a resin to produce a hollow fiber bundle;
(ii) introducing a gel precursor solution containing a nucleic acid probe into the hollow portion of each hollow fiber of the hollow fiber bundle;
(iii) performing a reaction of the gel precursor solution introduced into the hollow portion of each hollow fiber of the hollow fiber bundle to hold a gel containing the nucleic acid probe in the hollow portion of each hollow fiber;
(iv) slicing the hollow fiber bundle in a direction intersecting with the longitudinal direction of the hollow fibers to obtain the DNA chip;
(v) applying a composition for preventing drying of the gel, comprising: a polyhydric alcohol; and at least one selected from gelatin, collagen, carrageenan, pectin and agar, to the DNA chip or the portion of the gel of the DNA chip, or immersing the DNA chip in the composition for preventing drying of the gel; and
(vi) gelling the composition for preventing drying of the gel.

7. A method for preventing drying of a DNA chip, comprising the steps of:
applying a composition for preventing drying of a gel, comprising: a polyhydric alcohol; and at least one selected from gelatin, collagen, carrageenan, pectin and agar, to the DNA chip or the portion of the gel of the DNA chip, or immersing the DNA chip in the composition for preventing drying of the gel; and
gelling the composition for preventing drying of the gel.
